# EUROPEAN PATENT SPECIFICATION

(11) **EP 2 827 820 B1**
(45) Date of publication and mention of the grant of the patent: **10.05.2017**
(21) Application number: 13715514.9
(22) Date of filing: 19.03.2013
(51) Int. Cl.: A61F 13/40, A61L 15/44, A61L 15/46

(54) **HIGH ADHESION ANTIMICROBIAL SKIN PREP SOLUTIONS AND RELATED METHODS**
ANTIMIKROBIELLE HAUTVORBEREITUNGSLÖSUNGEN MIT HOHER HAFTKRAFT UND ZUGEHÖRIGE VERFAHREN
SOLUTIONS DE PRÉPARATION ANTIMICROBIENNE À HAUTE ADHÉRENCE POUR LA PEAU ET PROCÉDÉS ASSOCIÉS

(30) Priority: 19.03.2012 US 201261612573 P
(43) Date of publication of application: 28.01.2015
(73) Proprietor: entrotech life sciences, inc., San Francisco, CA 94158 (US)
(72) Inventor: MCGUIRE, James, E., Jr., Tiburon, CA 94920 (US)
(74) Representative: Laufhütte, Dieter
(86) International application number: PCT/US2013/033030
(87) International publication number: WO 2013/142537

(56) References cited:
- WO-A1-00/57933
- WO-A2-2010/080936
- US-A- 6 106 505
- US-A1- 2001 055 511

## Description

### BACKGROUND OF THE INVENTION

The present invention relates generally to antimicrobial skin prep solutions that facilitate improved adhesion of surgical drapes or medical dressings thereto.

Antimicrobial skin prep solutions are often applied to patient's skin before medical procedures. For example, 3M's DURAPREP Surgical Solution (an iodine povacrylex and isopropyl alcohol solution available from 3M Company of St. Paul, MN) is a well-known skin prep solution. As a further example, CHLORAPREP (2% chlorhexidine gluconate based on total weight of a solution containing 70% isopropyl alcohol and 30% water available from CareFusion of San Diego, CA) is also a well-known skin prep solution.

It is known that prepping a skin surface does not always completely eradicate bacteria. In contrast to surfaces covered by a sterile incise drape, a skin surface that has been "prepped" has only been disinfected, not sterilized. Even after prepping, bacteria regeneration continuously occurs on a skin surface. Further, skin prepping solutions are vulnerable to removal or neutralization by blood, irrigation fluids, exudates, and the like, which are commonly associated with surgical procedures. Thus, further antimicrobial efforts are generally engaged after a skin surface has been treated with a skin prepping solution.

One such antimicrobial effort involves application of a medical dressing, such as a surgical incise drape, to a skin surface. An "incise drape" is one where a surgical incision is made directly through the drape and into a patient. By definition, a skin surface covered by a sterile incise drape is bacteria-free (*i.e*., sterilized) at the beginning of a surgical procedure. Ideally, the skin surface remains bacteria-free during the procedure, resisting the transfer of bacteria from, for example, gloves, instruments, and sponges that may come in contact with not only the surgical wound but also the surrounding skin. Nevertheless, a drape's barrier and antimicrobial properties are typically effective only so long as the drape is securely attached to the skin. Many types of surgical drapes and medical dressings are known. Some include an adhesive layer for adhering the surgical drape to a patient's skin or other surface.

Adhesion of a drape to skin can be affected by a wide variety of considerations, including insufficient drying of any skin prepping solution and the type of any skin prepping solution applied to the skin before the drape is adhered thereto. For example, a 2007 technical brochure published by 3M Company (St. Paul, MN) describes test results illustrating that 3M's DURAPREP Surgical Solution (an iodine povacrylex and isopropyl alcohol solution available from 3M Company of St. Paul, MN) facilitates better adhesion of drapes to skin prepped therewith as compared to skin prepped with CHLORAPREP (2% chlorhexidine gluconate based on total weight of a solution containing 70% isopropyl alcohol and 30% water available from CareFusion of San Diego, CA). Indeed, skin prepped with DURAPREP is known to be tacky (which tackiness is not universally advantageous in that it is more susceptible to accumulation of unwanted particles and grime, for example, but it is advantageous from the perspective of promoting adherence of a surgical drape or other medical dressing to the prepped skin). One reason for this marked difference in adhesion may be due to use of iodine povacrylex (*i.e*., an iodine/acrylate copolymer) in the DURAPREP Surgical Solution made available by 3M Company. Notably absent from the marketplace, however, are conventional skin prep solutions similar to the iodine povacrylex-containing DURAPREP Surgical Solution, but containing chlorhexidine as the antimicrobial agent.

One reason for the absence of such a chlorhexidine-containing skin prep solution from the marketplace is the known difficulty in formulating stable chlorhexidine-containing solutions. For example, chlorhexidine is notoriously unstable and incompatible in solution form. Issues encountered with conventional attempts to provide stable chlorhexidine-containing skin prep solutions include unacceptable high levels of the genotoxic decomposition product, p-chloroaniline, and ineffective antimicrobial activity. These issues are described in, for example, PCT Patent Publication No. WO2011/061272, incorporated by reference herein in its entirety. Due to these known difficulties, those of ordinary skill in the art have not typically been motivated to, nor successful in any attempts to, incorporate additional components into chlorhexidine-containing skin prep solutions in order to improve adhesion thereof.

Chlorhexidine is typically present in skin prep solutions in its salt form (e.g., chlorhexidine gluconate, chlorhexidine acetate, etc.), which form is understood to generally require water for adequate solubilization. Chlorhexidine, a substituted diguanide, has a high degree of antimicrobial activity, low mammalian toxicity, and the ability to bind to the stratum corneum layer of skin and to mucous membranes. The bactericidal activity of chlorhexidine is much greater than that of monomeric biguanides. These unique characteristics make it particularly attractive as an active ingredient in antimicrobial applications.

Besides its use in specific medical dressings and skin antiseptics, the efficacy of chlorhexidine in providing antimicrobial protection is known further throughout the medical industry. For example, U.S. Patent No. 7,329,412 describes an antimicrobial catheter prepared by treating a polymeric catheter with a solution comprising a solvent and an antimicrobial mixture consisting essentially of chlorhexidine free base and a water-soluble chlorhexidine salt, wherein the weight ratio of chlorhexidine free base to water-soluble chlorhexidine salt in the solution is between 1:1 to 1:5. As background therein, duration of the antimicrobial efficacy of medical devices impregnated with chlorhexidine salts, such as chlorhexidine acetate, is discussed as being short lived. Further discussed is the fact that chlorhexidine free base is not generally known to be soluble in water or alcohol, which generally prevents it from being impregnated in sufficient amounts because of low solubility in a solvent system. Thus, the combination of chlorhexidine free base and a water-soluble chlorhexidine salt, at the particular ratios described therein, was found to provide improved antimicrobial effectiveness through an increased uptake of chlorhexidine into, increased retention of chlorhexidine in, and prolonged release of chlorhexidine from the medical device, while utilizing relatively low levels of chlorhexidine.

U.S. Patent No. 5,165,952 relates to medical articles employing chlorhexidine. When bulk-distributed in a medical article, chlorhexidine is described as being known to adversely affect certain characteristics of the article, such as tensile strength; and, when the medical article is formed of a plastic material, high temperatures often needed for extension of such plastic materials into the form of a medical article are described as potentially damaging the chlorhexidine within. Thus, chlorhexidine is both coated on and bulk-distributed throughout the medical articles according to the disclosure therein. Similarly, U.S. Patent No. 5,089,205 relates to incorporation of chlorhexidine free base or one of its salts into a medical device such as a glove. The chlorhexidine can be incorporated by both distribution and dipping processes.

Chlorhexidine, in its pure form and in its salt forms, is also discussed as being useful as a preservative and as an antimicrobial in compositions for oral hygiene. See, for example, U.S. Patent Publication No. 20050158252.

U.S. Patent Publication No. 20080026025 also discusses the use of chlorhexidine, specifically in water purification applications. As discussed therein, chlorhexidine is a 1,6-di(4-chlorophenyl-diguanido) hexane having the chemical formula: 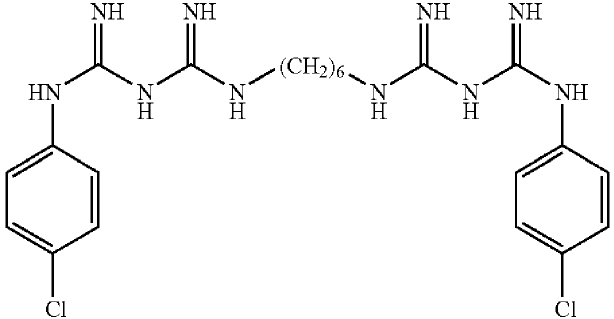 Chlorhexidine is discussed therein as having a high level of antibacterial activity, low mammalian toxicity, and a strong affinity for binding to skin and mucous membranes.

Improvements with respect to a surgical drape's or other medical dressing's adhesion to a variety of skin surfaces and its role in halting the spread of bacteria promoting staphylococcal infections are needed. Knowing that instructions for adequate adhesion of surgical drapes and other medical dressings are often not sufficiently followed by medical personnel, resulting in less than optimal adherence of surgical drapes and other medical dressings to insufficiently dried surfaces prepped with a skin prepping solution, alternatives for improved adhesion are desirable. In order to facilitate such improvements, improved antimicrobial skin prep solutions and methods for their use are desired.

### BRIEF SUMMARY OF THE INVENTION

Antimicrobial skin prep solutions of the invention comprise: a major amount of at least one organic solvent, wherein at least about 80% by weight based on total weight of the solvent comprises at least one fugitive organic solvent; an antimicrobially effective amount of at least one antimicrobial agent; and an amount of at least one adhesive effective to increase adhesion of surgical drapes and medical dressings to skin prepped with the antimicrobial skin prep solution, wherein the at least one adhesive is distinct from the at least one antimicrobial agent and is a liquid at room temperature. The adhesive is selected to be compatible with other components (*i.e*., antimicrobial agent and solvent) of the skin prep solution. Preferably, the adhesive is also skin-compatible. In one embodiment, the at least one adhesive functions as a hydrogel therein.

Advantageously, incorporation of the at least one adhesive according to the invention provides an increase in adhesion of surgical drapes and medical dressings to skin prepped with the antimicrobial skin prep solution of at least about 10 N/dm when tested according to a 90° Peel Adhesion Test Method described herein. According to a further embodiment, the increase in adhesion is at least about 20 N/dm when tested according to a 90° Peel Adhesion Test Method described herein. Preferably, adhesion of surgical drapes and medical dressings to skin prepped with the antimicrobial skin prep solution is at least about 25 N/dm when tested according to a 90° Peel Adhesion Test Method described herein. In further preferred embodiments, adhesion of surgical drapes and medical dressings to skin prepped with the antimicrobial skin prep solution is at least about 35 N/dm when tested according to a 90° Peel Adhesion Test Method described herein.

The adhesive may be based on (*i.e*., comprise) any suitable chemistry. According to a preferred embodiment, the adhesive is based on (meth)acrylate chemistry. In an exemplary embodiment, the at least one adhesive comprises polar functional groups.

In one embodiment, the at least one adhesive has a room temperature Brookfield viscosity of less than about 10,000 centiPoise. In a further embodiment, the at least one adhesive has a room temperature Brookfield viscosity of less than about 5,000 centiPoise. In yet a further embodiment, the at least one adhesive has a room temperature Brookfield viscosity of less than about 2,000 centiPoise. In still a further embodiment, the at least one adhesive has a room temperature Brookfield viscosity of the adhesive is about 400 centiPoise to about 1,500 centiPoise.

The antimicrobial agent may comprise any suitable chemistry and is present in an antimicrobially effective amount. According to a preferred embodiment, the antimicrobial agent comprises chlorhexidine. In an exemplary embodiment, the antimicrobial agent comprises chlorhexidine in its pure form (*i.e*., a chlorhexidine free base). In an exemplary embodiment, the antimicrobial agent comprises a chlorhexidine salt (*e.g*., chlorhexidine gluconate). An antimicrobially effective amount of the antimicrobial agent is at least about 2% by weight chlorhexidine salt in a preferred skin prep solution after it is applied to a substrate and dried according to the invention (*i.e*., a reduced skin prep solution).

In one embodiment, the amount of at least one adhesive effective to increase adhesion of surgical drapes and medical dressings to skin prepped with the antimicrobial skin prep solution is about 2% by weight of the skin prep solution. In a further embodiment, the amount of at least one adhesive effective to increase adhesion of surgical drapes and medical dressings to skin prepped with the antimicrobial skin prep solution is about 5.5% by weight of the skin prep solution.

In addition to improved skin prep solutions, applicators comprising the antimicrobial skin prep solution of the invention in one or multiple parts are also disclosed herein. Further disclosed is a method of disinfecting a skin surface, comprising steps of: applying the antimicrobial skin prep solution of the invention to the skin surface to provide a disinfected skin surface; and allowing the disinfected skin surface to dry. Also disclosed is the resulting coated substrate (*e.g*., skin) comprising the reduced skin prep solution. Advantageously, according to preferred embodiments, the skin surface has essentially no apparent tack after it is dry when disinfected according to the invention. Also disclosed is a method of sterilizing a skin surface, comprising steps of: applying the antimicrobial skin prep solution of the invention to the skin surface to provide a disinfected skin surface; allowing the disinfected skin surface to dry; and adhering a medical dressing, such as a surgical incise drape, to the disinfected skin surface. Similarly disclosed is the resulting coated substrate, where the reduced skin prep solution forms an interfacial tie layer between the substrate (*e.g*., skin) and the medical dressing.

### DETAILED DESCRIPTION OF THE INVENTION

Antimicrobial skin prep solutions comprise at least one adhesive according to the invention. Such solutions promote improved adherence properties when skin prepped with the solution is subsequently fitted with a surgical drape or other medical dressing. For example, one aspect of the invention is that such skin prep solutions dry relatively quickly as compared to conventional skin prep solutions. Advantageously, such quick drying decreases the chance that subsequent adherence of a surgical drape or other medical dressing will be negatively affected by medical personnel's failure to precisely follow dry time instructions and adhering the drape or dressing to an inadequately dried (*i.e*., dampened) surface on a patient's body recently treated with a skin prep solution.

According to another aspect of the invention, the antimicrobial skin prep solutions promote improved adherence properties when skin prepped with the solution is subsequently fitted with a surgical drape or other medical dressing by facilitating interfacial bonding. For example, antimicrobial skin prep solutions of the invention make a patient's body treated with the skin prep solution more chemically compatible with the drape or dressing subsequently fitted thereon, much as a tie layer or bonding layer functions within a laminate.

While skin prep solutions of the invention include at least one adhesive, they are formulated to have improved properties over conventional skin prep solutions, but without negative effects associated with adhesive tack. Tackiness is not universally advantageous in that it is more susceptible to accumulation of unwanted particles and grime. Preferably, after skin is prepped with skin prep solutions of the invention, it has essentially no apparent tack. In contrast, skin prepped with DURAPREP Surgical Solution (an iodine povacrylex and isopropyl alcohol solution available from 3M Company of St. Paul, MN) is known to have apparent tack as discussed above. Apparent tack is measurable by, for example, lightly pressing a surgical glove against the prepped surface to determine whether it sticks thereto (*e.g*., as evidenced by stringing or legging of the material when pulling away the glove).

According to one embodiment, incorporation of at least one adhesive into a skin prep solution according to the invention provides an increase in adhesion of surgical drapes and medical dressings to skin prepped with the antimicrobial skin prep solution of at least about 10 N/dm when tested according to a 90° Peel Adhesion Test Method described herein. In a further embodiment, the increase in adhesion due to incorporation of the at least one adhesive is at least about 20 N/dm when tested according to the 90 Peel Adhesion Test Method described herein. Preferably, adhesion of surgical drapes and medical dressings to skin prepped with the antimicrobial skin prep solution is at least about 25 N/dm when tested according to a 90° Peel Adhesion Test Method described herein. In further preferred embodiments, adhesion of surgical drapes and medical dressings to skin prepped with the antimicrobial skin prep solution is at least about 35 N/dm when tested according to a 90° Peel Adhesion Test Method described herein.

### Antimicrobial Agent

Any suitable antimicrobial agent may be included in skin prep solutions according to the invention. Advantageously, the present invention provides a solution to the known problem of obtaining chlorhexidine-containing skin prep solutions with improved adhesion. For example, preferred skin prep solutions of the invention are chlorhexidine-containing. Chlorhexidine-containing skin prep solutions comprise an antimicrobially effective amount of chlorhexidine in its pure form (*i.e*., as a free base) and/or in the form of at least one chlorhexidine salt. For example, chlorhexidine free base is commercially available from Sigma-Aldrich Corp. (St. Louis, MO) as a solution of 98% chlorhexidine.

Further exemplary skin prep solutions of the invention comprise an antimicrobially effective amount of chlorhexidine in the form of at least one chlorhexidine salt. Suitable counterions for chlorhexidine include, but are not limited to, dihydrochloride, methosulfate, lactate, gluconate, acetate, diacetate, and the like. An exemplary chlorhexidine salt is chlorhexidine gluconate (CHG). For example, chlorhexidine digluconate is commercially available from Sigma-Aldrich Corp. (St. Louis, MO) as a solution of solution of 20% by weight chlorhexidine digluconate in water. It is to be understood herein that chlorhexidine gluconate as used herein includes chlorhexidine digluconate and the like. The same understanding applies to other chlorhexidine-containing antimicrobial agents in their salt form.

An anti microbially effective amount of an antimicrobial agent is present in skin prep solutions of the invention. In one embodiment, an antimicrobially effective amount of chlorhexidine is present in skin prep solutions of the invention. In an exemplary embodiment, at least about 1 % by weight chlorhexidine salt is present in the reduced skin prep solution. In another exemplary embodiment, at least about 2% by weight chlorhexidine salt is present in the reduced skin prep solution. In another exemplary embodiment, at least about 3% by weight chlorhexidine salt is present in the reduced skin prep solution.

### Adhesive

Improved skin prep solutions of the invention contain at least one adhesive that is distinct from the antimicrobial agent. The adhesive component of skin prep solutions of the invention is a liquid (*i.e*., it is a fluid that flows without application of external force) at room temperature (*i.e*., about 22°C to about 25°C) in its neat form (*i.e*., 100% non-volatile, also referred to herein as essentially free of fugitive solvent).

The adhesive is selected to have a relatively short length of polymer chain, which facilitates obtainment of an adhesive having a viscosity suitable for use in the present skin prep solutions. In one embodiment, room temperature Brookfield viscosity of the adhesive is less than about 10,000 centiPoise. In another embodiment, room temperature Brookfield viscosity of the adhesive is less than about 5,000 centiPoise. In yet another embodiment, room temperature Brookfield viscosity of the adhesive is less than about 2,000 centiPoise. In still a further embodiment, room temperature Brookfield viscosity of the adhesive is less than about 1,500 centiPoise. In an exemplary embodiment, room temperature Brookfield viscosity of the adhesive is about 50 centiPoise to about 1,500 centiPoise. In another exemplary embodiment, room temperature Brookfield viscosity of the adhesive is about 400 centiPoise to about 1,500 centiPoise. Viscosity is measurable according to techniques well known to those of ordinary skill in the art and may be measured using, for example, a Brookfield rotational viscometer such as those available from Cole-Parmer (Vernon Hills, IL). Viscosity measurements indicated herein are of the adhesive in its neat form (*i.e*., 100% non-volatile), without the presence of viscosity-reducing solvents.

Adhesives of the invention are based on (*i.e*., comprise) any suitable polymer chemistry. With adjustments to their molecular weight such that the adhesive is a liquid at room temperature, many conventional adhesive chemistries can be adapted successfully according to the present invention. For example, adhesives based on chemistries known to be useful as pressure sensitive adhesives and described in U.S. Patent Nos. 2,884,126 and 4,181,752, incorporated by reference in their entirety herein, can be adapted for use herein. Pressure sensitive adhesives are well known to those of ordinary skill in the art to possess properties including the following: (1) aggressive and permanent tack, (2) adherence with no more than finger pressure, (3) sufficient ability to hold onto an adherend, and (4) sufficient cohesive strength. Materials that have been found to function well as pressure sensitive adhesives are polymers designed and formulated to exhibit the requisite viscoelastic properties resulting in a desired balance of tack, peel adhesion, and shear holding power.

In contrast, however, preferred adhesives of the invention do not function as a tacky adhesive, such as pressure sensitive adhesives, in the form in which they are used. That is, adhesives of the invention are liquids at room temperature (without requiring use of viscosity-reducing fugitive solvents), as compared to many conventional tacky adhesives that are solids at room temperature, in the form in which they are used after application to a surface and drying (*i.e*., after removal of any viscosity-reducing fugitive solvents). Given their liquid form, adhesives of the invention lack sufficient cohesive strength to impart tack, whether present without other components or formulated into a skin prep solution of the invention, before application to a surface. Even after application of the adhesive to a surface and upon drying, whether present without other components or formulated into a skin prep solution of the invention, adhesives of the invention lack sufficient cohesive strength to impart tack to the surface. In contrast, film-forming adhesives generally exhibit tack, at least during the process of drying after application to a surface, due to interaction of typically more densely packed polymer chains therein.

The adhesive is selected such that it is compatible with other components (*i.e*., antimicrobial agent and solvent) of the skin prep solution. By "compatible" is meant that the component does not cause a significant and undesired change in functional properties when brought into contact with each of the other components. Compatibility is evidenced by the absence of phase separation, viewable by the unaided human eye, between components of the skin prep solution. Generally and surprisingly, components of the skin prep solution remain compatible for the duration of the solution's shelf life.

According to another preferred aspect of the invention, the adhesive is selected such that it is compatible with skin on which skin prep solutions of the invention are used. That is, the at least one adhesive comprises a skin-compatible adhesive. "Skin-compatible adhesives" of the invention are those that do not lead to statistically significant skin discomfort or skin color change during contact between the adhesive and skin for a period of six hours. Particularly preferred adhesives are those fulfilling requirements to be designated as general regarded as safe - *i.e*., GRAS - according to the U.S. Food and Drug Administration standards.

As the adhesive is a distinct component of the skin prep solution, the antimicrobial agent is generally not reacted with or incorporated into the polymer on which the adhesive is based (*i.e*., the antimicrobial agent is not bonded covalently or via charge transfer complex, the latter of which is associated with iodine formulations such as 3M's DURAPREP Surgical Solution (an iodine povacrylex and isopropyl alcohol solution available from 3M Company of St. Paul, MN).

As the adhesive is a distinct component of the skin prep solution, the adhesive is not required to be based on water-soluble monomers (*e.g*., N-vinyl lactam monomers), which water-soluble monomers are conventionally required when formulating certain types of skin prep solutions (*i.e*., those associated with complexed iodine formulations). Advantageously, when use of water-soluble monomers is minimized, adhesives with a higher degree of hydrophobicity (*i.e*., essentially no water solubility) are obtained. Accordingly, in one embodiment, the adhesive is essentially free of water-soluble monomers. That is, the adhesive is preferably based on less than about 1% by weight of, more preferably no, water-soluble monomers based on total monomer weight. In an exemplary embodiment, chemistry of adhesives of the invention comprises (meth)acrylate-based polymers, including methacrylates and/or acrylates. In a further embodiment, adhesives of the invention include those based on acrylate and urethane/acrylate chemistries. Suitable acrylic copolymer adhesive solutions for use in the present invention are also commercially available from entrochem, inc. (Columbus, OH) under the trade designations, eca-373 and eca-436. By minimizing or eliminating water-solubility of the adhesive, which property is promoted by minimizing or eliminating use of water-soluble monomers when forming the same, antimicrobial properties of skin treated with skin prep solutions of the invention was found to be more resistant to degradation with respect to the beneficial antimicrobial properties imparted by the reduced skin prep solution when, for example, contacted with water-based bodily fluids such as blood, perspiration, and the like.

Further preferable are homopolymeric adhesives, particularly homopolymers based on water-insoluble monomers, which are generally understood to have the same solubility along their length. Again, by minimizing or eliminating water-solubility of the adhesive, antimicrobial properties of skin treated with skin prep solutions of the invention was found to be more resistant to degradation with respect to the beneficial antimicrobial properties imparted by the reduced skin prep solution when, for example, contacted with water-based bodily fluids such as blood, perspiration, and the like. An exemplary homopolymeric adhesive is based on 100% iso-octyl acrylate.

In one embodiment, adhesives of the invention include polar functional groups. According to one aspect of this embodiment, an adhesive is modified to function as a hydrogel in skin prep solutions of the invention. In one variation of this embodiment, polymer moieties comprising at least one hydrophilic moiety and at least one hydrophobic moiety are incorporated into the adhesive. In another variation of this embodiment, the polar moieties are incorporated into the backbone of the polymer chain forming the adhesive. According to an exemplary embodiment, the polar moieties are incorporated at opposite ends of the polymer chain forming the adhesive. An exemplary adhesive including polar functional groups is formed from 90% iso-octyl acrylate and 10% acrylic acid based on total weight of the adhesive.

An amount of at least one adhesive effective to increase adhesion of surgical drapes and medical dressings to skin prepped therewith is present in skin prep solutions of the invention. However, preferably the adhesive is present in a small enough amount that the resulting skin prep solution (*i.e*., the reduced skin prep solution) has essentially no apparent tack. For example, the at least one adhesive may be present in the skin prep solution in an amount of about 1% to about 10% by weight of the skin prep solution. In an exemplary embodiment, an effective amount of the at least one adhesive is at least about 2% by weight of the skin prep solution. In a further embodiment, an effective amount of the at least one adhesive is at least about 3% by weight of the skin prep solution. In a yet further embodiment, an effective amount of the at least one adhesive is at least about 4% by weight of the skin prep solution. In still a further embodiment, an effective amount of the at least one adhesive is at least about 5.5% by weight of the skin prep solution.

### Solvent

Skin prep solutions of the invention are formulated using a major amount of at least one organic solvent into which the at least one antimicrobial agent and the at least one adhesive are formulated. This major amount of at least one organic solvent is understood to be a major amount of at least one fugitive solvent. "Fugitive solvents" are those solvents expected to volatilize completely at room temperature after application of the desired skin prep solution to a surface and drying of the same. In contrast, "non-fugitive solvents" are those solvents included in the skin prep solution that are expected to remain in the skin prep solution at room temperature even after volatilization of the fugitive solvents when the skin prep solution is applied to a surface and dried. The evaporation rate of non-fugitive solvents is slower than that of fugitive solvents conventionally used in skin prep solutions. As used throughout the description of the invention, "dry," "drying," and "dried" refers to removal of essentially all fugitive solvents from the skin prep solution such that it is non-volatile (*i.e*., a "reduced skin prep solution"). Unless otherwise specified, "solvent" as used herein means "fugitive solvent."

Conventional skin prep solutions are typically based on fugitive solvents of 70% isopropyl alcohol (IPA) and 30% water by weight based on total weight of the solvent. In contrast, however, preferred skin prep solutions of the invention are formulated using a major amount of at least one organic solvent, wherein at least about 80% by weight based on total weight of the solvent, more preferably all of the solvent, comprises at least one fugitive organic solvent (*i.e*., 100% fugitive organic solvent). More preferably, skin prep solutions of the invention are based essentially only on fugitive, organic, water-soluble solvent (*e.g*., alcohols, such as ethanol, methanol, N-propanol, and IPA, and esters thereof, such as ethyl acetate).

Recognize, however, that solvents other than the major amount of at least one organic solvent may be present in the skin prep solution due to their inclusion with individual components added to form the skin prep solution. For example, antimicrobial agents (*e.g*., chlorhexidine-containing antimicrobial agents) often contain water as a solvent for use in formulation. Minor amounts of water may, thus, be present in skin prep solutions formulated therefrom. In any event, the formulated skin prep solution is preferably essentially free of inorganic solvent (*e.g*., water). That is, skin prep solutions of the invention preferably comprise less than about 0.3% by weight, more preferably less than about 0.003% by weight, water based on total weight of the skin prep solution. Reduction or elimination of water advantageously decreases drying time of a skin surface treated with the skin prep solution.

An exemplary solvent useful in formulating skin prep solutions of the invention is essentially pure IPA. By substantially eliminating water from the solvent, solubility of preferred chlorhexidine-containing antimicrobial agents within the skin prep solution was improved and use of advantageous adhesives was enabled.

### Methods for Preparation

Skin prep solutions of the invention are readily preparable according to methodology known to those of ordinary skill in the art. Exemplary methods for preparation of skin prep solutions of the invention are described in the Examples section herein. Exemplary skin prep solutions according to the invention are also commercially available from entrochem, inc.

### Application

Formulated skin prep solutions can be applied to a surface using any suitable technology as known to those of ordinary skill in the art. For example, skin prep solutions of the invention may be applied to a surface using a liquid applicator to sufficiently wet the surface. The applied skin prep solution is then dried. No extra steps or equipment are necessary to efficiently dry skin prep solutions applied to a surface. In contrast, many conventional adhesives require ventilation hoods and forced air and/or heat to adequately dry solvent therefrom.

Once dried, skin prep solutions of the invention generally form a reduced skin prep solution coating having a viscous, liquid consistency on the prepped surface. In contrast, 3M's DURAPREP Surgical Solution (an iodine povacrylex and isopropyl alcohol solution available from 3M Company of St. Paul, MN) dries to a film having a solid consistency. As further contrasted, CHLORAPREP (2% chlorhexidine gluconate based on total weight of a solution containing 70% isopropyl alcohol and 30% water available from CareFusion of San Diego, CA) dries to a residue having a chalky consistency.

Reduced skin prep solutions of the invention function as interfacial tie layers that facilitate interfacial bonding and enhanced compatibility of the prepped surface when brought into contact with a surgical drape or other medical dressing. The liquid consistency of reduced skin prep solutions of the invention enables the resulting thin (*i.e*., preferably less than about 25 gsm, more preferably less than about 12.5 gsm, even more preferably less than about 5 gsm, and most preferably less than about 2.5 gsm) interfacial tie layer to be quickly adsorbed to the surface of the surgical drape or other medical dressing into which it is brought into contact. The liquid consistency further facilitates relatively quickly wet out of the contacted surgical drape or other medical dressing and adherence of the same to the skin. Advantageously, skin prep solutions of the invention facilitate relatively quick adhesion build-up when used as such. This improves efficiency and helps compensate in situations where instructions for adequate adhesion of surgical drapes and other medical dressings are not sufficiently followed by medical personnel.

Exemplary embodiments and applications of the invention are described in the following non-limiting examples.

### EXAMPLES

### Test Methods

Prior to testing, all skin prep solutions were conditioned for about 2-4 hours in a constant temperature (23°C.), constant humidity (50% relative humidity) environment. Tests were performed under the same atmospheric conditions.

### 90° Peel Adhesion Test Method

Peel adhesion is the force required to remove an adhesive-coated, flexible sheet material from a surface. Peel adhesion is measured at a specific angle and rate of removal. In the following examples, this peel adhesion force is expressed in Newtons/decimeter width (N/dm) and ounces/inch width (oz/in) of the adhesive-coated, flexible sheet material. Peel adhesion forces measured throughout are initial peel adhesion forces taken at about two minutes dwell time, unless indicated to the contrary. These initial peel adhesion forces may not be indicative of aged peel adhesion forces that can be obtained.

All tests were run using a 3M™ loban™ 2 Antimicrobial Incise Drape available from 3M Company (St. Paul, MN) as the adhesive-coated, flexible sheet material. In order to prevent stretching, a 2.5mil backing film (tape with adhesive) was applied to the back side of the adhesive-coated, flexible sheet material (*i.e*., the non-adhesive side). A liberal amount of the skin prep solution to be tested, except in the case of a control where no skin prep solution was used, was applied to skin on the underside of a Caucasian male's forearm and allowed to dry for about 2-3 minutes. A strip (2.54-cm wide) of the adhesive-coated, flexible sheet material/backing film laminate was applied to a horizontal surface on the underside of the prepped forearm so that at least 12 lineal centimeters of firm contact resulted. One pass with a 2-kilogram hard rubber roller was used to apply the strip. The free end of the strip was positioned so that the angle of removal was 90°, and it was attached to the adhesion tester clamp. The top side of the forearm remained resting against a metal plate that was capable of moving the plate away from the clamp at a constant rate of 3.2 meters/minute and a peel adhesion value was recorded as the strip was peeled from the underside of the forearm.

The peel adhesion test was repeated three times for each example at approximately the exact same location on the forearm. In general, the first value reported was lower than subsequent values reported for an example because excess oil and cells are peeled away from the skin initially as compared to that associated with later peels from the same site on the skin. The mean value for each example was then calculated, excluding the first value reported. Each of these values is reported in Table 1 below.

### Example 1

Into a glass jar at room temperature and humidity, 0.10 gram of a solution of 20% by weight chlorhexidine digluconate (chlorhexidine digluconate solution available from Sigma-Aldrich of St. Louis, MO) and 80% by weight water was added. 60.90 grams of isopropyl alcohol (2-propanol available from Sigma-Aldrich of St. Louis, MO) was continuously stirred into the solution for about 2-3 minutes. Then, 2.51 grams of an adhesive available from entrochem, inc. (Columbus, OH) under the trade designation, eca-373, was continuously stirred into the solution for about 4-5 minutes to form an improved skin prep solution according to the invention. The overall weight percentage of the adhesive in the skin prep solution thus formed was 4.0% based on weight of the skin prep solution and the overall weight percentage of the chlorhexidine digluconate in the skin prep solution was 0.03% based on weight of the skin prep solution.

After application to a substrate and upon drying, the overall weight percentage of the chlorhexidine digluconate in the skin prep was 0.8% based on weight of the adhesive. This coated and reduced skin prep solution was then tested according to the 90° Peel Adhesion Test Method described above. Results are reported in Table 1 below.

### Example 2

Into a glass jar at room temperature and humidity, 0.13 gram of a solution of 20% by weight chlorhexidine digluconate (chlorhexidine digluconate solution available from Sigma-Aldrich of St. Louis, MO) and 80% by weight water was added. 65.5 grams of isopropyl alcohol (2-propanol available from Sigma-Aldrich of St. Louis, MO) was continuously stirred into the solution for about 2-3 minutes. Then, 3.99 grams of an adhesive available from entrochem, inc. (Columbus, OH) under the trade designation, eca-373, was continuously stirred into the solution for about 4-5 minutes to form an improved skin prep solution according to the invention. The overall weight percentage of the adhesive in the skin prep solution thus formed was 5.7% based on weight of the skin prep solution and the overall weight percentage of the chlorhexidine digluconate in the skin prep solution was 0.04% based on weight of the skin prep solution.

After application to a substrate and upon drying, the overall weight percentage of the chlorhexidine digluconate in the skin prep was 0.7% based on weight of the adhesive. This coated and reduced skin prep solution was then tested according to the 90° Peel Adhesion Test Method described above. Results are reported in Table 1 below.

### Example 3

Into a glass jar at room temperature and humidity, 0.27 gram of a solution of 20% by weight chlorhexidine digluconate (chlorhexidine digluconate solution available from Sigma-Aldrich of St. Louis, MO) and 80% by weight water was added. 65.5 grams of isopropyl alcohol (2-propanol available from Sigma-Aldrich of St. Louis, MO) was continuously stirred into the solution for about 2-3 minutes. Then, 3.99 grams of an adhesive available from entrochem, inc. (Columbus, OH) under the trade designation, eca-373, was continuously stirred into the solution for about 4-5 minutes to form an improved skin prep solution according to the invention. The overall weight percentage of the adhesive in the skin prep solution thus formed was 5.7% based on weight of the skin prep solution and the overall weight percentage of the chlorhexidine digluconate in the skin prep solution was 0.08% based on weight of the skin prep solution.

After application to a substrate and upon drying, the overall weight percentage of the chlorhexidine digluconate in the skin prep was 1.4% based on weight of the adhesive. This coated and reduced skin prep solution was then tested according to the 90° Peel Adhesion Test Method described above. Results are reported in Table 1 below.

### Example 4

Into a glass jar at room temperature and humidity, 0.46 gram of a solution of 20% by weight chlorhexidine digluconate (chlorhexidine digluconate solution available from Sigma-Aldrich of St. Louis, MO) and 80% by weight water was added. 67.0 grams of isopropyl alcohol (2-propanol available from Sigma-Aldrich of St. Louis, MO) was continuously stirred into the solution for about 2-3 minutes. Then, 4.01 grams of an adhesive available from entrochem, inc. (Columbus, OH) under the trade designation, eca-373, was continuously stirred into the solution for about 4-5 minutes to form an improved skin prep solution according to the invention. The overall weight percentage of the adhesive in the skin prep solution thus formed was 5.6% based on weight of the skin prep solution and the overall weight percentage of the chlorhexidine digluconate in the skin prep solution was 0.13% based on weight of the skin prep solution.

After application to a substrate and upon drying, the overall weight percentage of the chlorhexidine digluconate in the skin prep was 2.3% based on weight of the adhesive. This coated and reduced skin prep solution was then tested according to the 90° Peel Adhesion Test Method described above. Results are reported in Table 1 below.

### Example 5

Into a glass jar at room temperature and humidity, 4.21 grams of a solution of 20% by weight chlorhexidine digluconate (chlorhexidine digluconate solution available from Sigma-Aldrich of St. Louis, MO) and 80% by weight water was added. 34.60 grams of isopropyl alcohol (2-propanol available from Sigma-Aldrich of St. Louis, MO) was continuously stirred into the solution for about 2-3 minutes. Then, 2.38 grams of an adhesive available from entrochem, inc. (Columbus, OH) under the trade designation, eca-373, was continuously stirred into the solution for about 4-5 minutes to form an improved skin prep solution according to the invention. The overall weight percentage of the adhesive in the skin prep solution thus formed was 5.8% based on weight of the skin prep solution, and the overall weight percentage of the chlorhexidine digluconate in the skin prep solution was 2.0% based on weight of the skin prep solution.

After application to a substrate and upon drying, the overall weight percentage of the chlorhexidine digluconate in the skin prep was 26.1% based on weight of the adhesive. This coated and reduced skin prep solution was then tested according to the 90° Peel Adhesion Test Method described above. Results are reported in Table 1 below.

### Example 6

Into a glass jar at room temperature and humidity, 0.09 gram of a solution of 98% by weight chlorhexidine (available from Sigma-Aldrich of St. Louis, MO) and 2% by weight water was dissolved in 5.0 grams of ethyl acetate available from Sigma-Aldrich (St. Louis, MO). Then, 57.0 grams of isopropyl alcohol (2-propanol available from Sigma-Aldrich of St. Louis, MO) was continuously stirred into the solution for about 2-3 minutes. Then, 3.80 grams of an adhesive available from entrochem, inc. (Columbus, OH) under the trade designation, eca-436, was continuously stirred into the solution for about 4-5 minutes to form an improved skin prep solution according to the invention. The overall weight percentage of the adhesive in the skin prep solution thus formed was 3.2% based on weight of the skin prep solution and the overall weight percentage of the chlorhexidine in the skin prep solution was 0.134% based on weight of the skin prep solution.

After application to a substrate and upon drying, the overall weight percentage of the chlorhexidine in the skin prep was 3.6% based on weight of the adhesive. This coated and reduced skin prep solution was then tested according to the 90° Peel Adhesion Test Method described above. Results are reported in Table 1 below.

### Example 7

Into a glass jar at room temperature and humidity, 0.08 gram of a solution of 98% by weight chlorhexidine (available from Sigma-Aldrich of St. Louis, MO) and 2% by weight water was added was dissolved in 5.1 grams of ethyl acetate available from Sigma-Aldrich (St. Louis, MO). Then, 48.1 grams of isopropyl alcohol (2-propanol available from Sigma-Aldrich of St. Louis, MO) was continuously stirred into the solution for about 2-3 minutes. Then, 2.39 grams of an adhesive available from entrochem, inc. (Columbus, OH) under the trade designation, eca-436, was continuously stirred into the solution for about 4-5 minutes to form an improved skin prep solution according to the invention. The overall weight percentage of the adhesive in the skin prep solution thus formed was 4.3% based on weight of the skin prep solution and the overall weight percentage of the chlorhexidine in the skin prep solution was 0.141% based on weight of the skin prep solution.

After application to a substrate and upon drying, the overall weight percentage of the chlorhexidine in the skin prep was 3.3% based on weight of the adhesive. This coated and reduced skin prep solution was then tested according to the 90° Peel Adhesion Test Method described above. Results are reported in Table 1 below.

### Example 8

Into a glass jar at room temperature and humidity, 0.11 gram of a solution of 98% by weight chlorhexidine (available from Sigma-Aldrich of St. Louis, MO) and 2% by weight water was dissolved in 5.0 grams of ethyl acetate available from Sigma-Aldrich (St. Louis, MO). Then, 57.0 grams of isopropyl alcohol (2-propanol available from Sigma-Aldrich of St. Louis, MO) was continuously stirred into the solution for about 2-3 minutes. Then, 2.46 grams of an adhesive available from entrochem, inc. (Columbus, OH) under the trade designation, eca-436, was continuously stirred into the solution for about 4-5 minutes to form an improved skin prep solution according to the invention. The overall weight percentage of the adhesive in the skin prep solution thus formed was 5.8% based on weight of the skin prep solution and the overall weight percentage of the chlorhexidine in the skin prep solution was 0.167% based on weight of the skin prep solution.

After application to a substrate and upon drying, the overall weight percentage of the chlorhexidine in the skin prep was 2.9% based on weight of the adhesive. This coated and reduced skin prep solution was then tested according to the 90° Peel Adhesion Test Method described above. Results are reported in Table 1 below.

### Example 9

Into a glass jar at room temperature and humidity, 0.12 gram of a solution of 98% by weight chlorhexidine (available from Sigma-Aldrich of St. Louis, MO) and 2% by weight water was dissolved in 5.0 grams of ethyl acetate available from Sigma-Aldrich (St. Louis, MO). Then, 50.0 grams of isopropyl alcohol (2-propanol available from Sigma-Aldrich of St. Louis, MO) was continuously stirred into the solution for about 2-3 minutes. Then, 4.74 grams of an adhesive available from entrochem, inc. (Columbus, OH) under the trade designation, eca-436, was continuously stirred into the solution for about 4-5 minutes to form an improved skin prep solution according to the invention. The overall weight percentage of the adhesive in the skin prep solution thus formed was 8.3% based on weight of the skin prep solution and the overall weight percentage of the chlorhexidine in the skin prep solution was 0.141% based on weight of the skin prep solution.

After application to a substrate and upon drying, the overall weight percentage of the chlorhexidine in the skin prep was 2.5% based on weight of the adhesive. This coated and reduced skin prep solution was then tested according to the 90° Peel Adhesion Test Method described above. Results are reported in Table 1 below.

### Comparative Example C1 - CHLORAPREP

CHLORAPREP (2% chlorhexidine gluconate based on total weight of a solution containing 70% isopropyl alcohol and 30% water available from CareFusion of San Diego, CA) was tested according to the 90° Peel Adhesion Test Method described above. Results are reported in Table 1 below.

### Comparative Example C2 - DURAPREP

DURAPREP Surgical Solution (an iodine povacrylex and isopropyl alcohol solution available from 3M Company of St. Paul, MN) was tested according to the 90° Peel Adhesion Test Method described above. Results are reported in Table 1 below.

### Comparative Example C3 - No Skin Prep

A control, where skin was not prepped with a solution, was tested according to the 90° Peel Adhesion Test Method described above. Results are reported in Table 1 below.

### Comparative Example C4

Into a glass jar at room temperature and humidity, 0.12 gram of a solution of 20% by weight chlorhexidine digluconate (chlorhexidine digluconate solution available from Sigma-Aldrich of St. Louis, MO) and 80% by weight water was added. 65.75 grams of isopropyl alcohol (2-propanol available from Sigma-Aldrich of St. Louis, MO) was then continuously stirred into the solution for about 2-3 minutes. The overall weight percentage of the chlorhexidine digluconate in the skin prep solution was 0.04% based on weight of the skin prep solution.

After application to a substrate and upon drying, this skin prep solution was then tested according to the 90° Peel Adhesion Test Method described above. Results are reported in Table 1 below.

**Table 1**

| Skin Prep Solution | 90° Peel Adhesion - N/dm (oz/in) | |
|---|---|---|
| Example 1 | 24.98 (22.82) | |
| | 37.28 (34.06) | Mean = 38.97 (35.61) |
| | 40.66 (37.15) | |
| Example 2 | 43.73 (39.95) | |
| | 45.37 (41.45) | Mean = 47.19 (43.11) |
| | 49.00 (44.77) | |
| Example 3 | 25.92 (23.68) | |
| | 34.57 (31.58) | Mean = 36.66 (33.49) |
| | 38.75 (35.40) | |
| Example 4 | 28.67 (26.19) | |
| | 30.52 (27.88) | Mean = 37.42 (34.19) |
| | 44.33 (40.50) | |
| Example 5 | 30.40 (27.77) | |
| | 35.78 (32.69) | Mean = 41.82 (38.21) |
| | 47.85 (43.72) | |
| Example 6 | 21.74 (19.86) | |
| | 27.17 (24.82) | Mean = 27.43 (25.06) |
| | 27.69 (25.30) | |
| Example 7 | 24.69 (22.56) | |
| | 28.46 (26.00) | Mean = 29.85 (27.28) |
| | 31.25 (28.55) | |
| Example 8 | 31.21 (28.51) | |
| | 34.61 (31.62) | Mean = 34.10 (31.16) |
| | 33.59 (30.69) | |
| Example 9 | 25.73 (23.51) | |
| | 29.38 (26.84) | Mean = 29.75 (27.18) |
| | 30.11 (27.51) | |
| Comparative Example C1 | 18.77 (17.15) | |
| | 20.61 (18.83) | Mean = 22.30 (20.38) |
| | 23.99 (21.92) | |
| Comparative Example C2 | 18.39 (16.80) | |
| | 23.34 (21.32) | Mean = 33.97 (31.03) |
| | 44.59 (40.74) | |
| Comparative Example C3 | 15.03 (13.73) | |
| | 21.54 (19.68) | Mean = 18.12 (16.56) |
| | 14.70 (13.43) | |
| Comparative Example C4 | 12.09 (11.05) | |
| | 20.15 (18.41) | Mean = 21.64 (19.77) |
| | 23.12 (21.12) | |

## Claims

1. An antimicrobial skin prep solution comprising:
a major amount of at least one organic solvent, wherein at least 80% by weight based on total weight of the solvent comprises at least one fugitive organic solvent;
an antimicrobially effective amount of at least one chlorhexidine-containing antimicrobial agent; and
an amount of at least one adhesive effective to increase adhesion of surgical drapes and medical dressings to skin prepped with the antimicrobial skin prep solution,
wherein the at least one adhesive is distinct from the at least one antimicrobial agent and is a liquid at room temperature.

2. The antimicrobial skin prep solution of claim 1, wherein the amount of at least one adhesive effective to increase adhesion of surgical drapes and medical dressings to skin prepped with the antimicrobial skin prep solution is 1% to 10% by weight of the skin prep solution.

3. The antimicrobial skin prep solution of any of the foregoing claims, wherein the at least one adhesive has a room temperature Brookfield viscosity of 400 centiPoise to 1,500 centiPoise.

4. The antimicrobial skin prep solution of any of the foregoing claims, wherein the at least one adhesive is based on (meth)acrylate chemistry.

5. The antimicrobial skin prep solution of any of the foregoing claims, wherein the at least one adhesive is based on monomers that are essentially free of watersoluble monomers.

6. The antimicrobial skin prep solution of any of the foregoing claims, wherein the at least one adhesive is a homopolymer.

7. The antimicrobial skin prep solution of any of the foregoing claims, wherein the antimicrobial agent comprises chlorhexidine free base.

8. The antimicrobial skin prep solution of any of the foregoing claims, wherein the antimicrobial agent comprises a chlorhexidine salt.

9. The antimicrobial skin prep solution of any of the foregoing claims, wherein at least 2% by weight chlorhexidine salt is present in the skin prep solution.

10. The antimicrobial skin prep solution of any of the foregoing claims, wherein the at least one adhesive comprises polar functional groups.

11. The antimicrobial skin prep solution of any of the foregoing claims, wherein the at least one adhesive provides an increase in adhesion of surgical drapes and medical dressings to skin prepped with the antimicrobial skin prep solution of at least 10 N/dm when tested according to a 90° Peel Adhesion Test Method described herein.

12. The antimicrobial skin prep solution of any of the foregoing claims, wherein the at least one adhesive is skin-compatible.

13. An applicator comprising the antimicrobial skin prep solution of any of the foregoing claims in one or multiple parts.

14. A coated substrate comprising a reduced skin prep solution formed by:
coating the skin prep solution of any of the foregoing claims thereon; and,
drying the coating to form the reduced skin prep solution,
wherein the reduced skin prep solution has a liquid consistency.

15. The coated substrate of claim 14, further comprising a medical dressing adhered thereto such that the reduced skin prep solution forms an interfacial tie layer between the substrate and the medical dressing.

## Patentansprüche

1. Antimikrobielle Hautvorbereitungslösung, umfassend:
eine größere Menge mindestens eines organischen Lösungsmittels, wobei mindestens 80 Gew.-% beruhend auf dem Gesamtgewicht des Lösungsmittels aus mindestens einem flüchtigen organischen Lösungsmittel bestehen;
eine antimikrobiell wirksame Menge mindestens eines chlorhexidinhaltigen antimikrobiellen Wirkstoffs; und
eine Menge mindestens eines Klebstoffs, welcher wirksam ist, um ein Anhaften von Operationsabdecktüchern und medizinischen Verbandstoffen an mit antimikrobieller Hautvorbereitungslösung vorbereiteter Haut zu verbessern,
wobei der mindestens eine Klebstoff verschieden von dem mindestens einen antimikrobiellen Wirkstoff ist und bei Raumtemperatur eine Flüssigkeit ist.

2. Antimikrobielle Hautvorbereitungslösung nach Anspruch 1, wobei die Menge mindestens eines Klebstoffs, welcher wirksam ist, um ein Anhaften von Operationsabdecktüchern und medizinischen Verbandstoffen an mit antimikrobieller Hautvorbereitungslösung vorbereiteter Haut zu verbessern, 1 - 10 Gew.-% der Hautvorbereitungslösung beträgt.

3. Antimikrobielle Hautvorbereitungslösung nach einem der vorhergehenden Ansprüche, wobei der mindestens eine Klebstoff eine Brookfield-Viskosität bei Raumtemperatur von 400 Centipoise bis 1.500 Centipoise aufweist.

4. Antimikrobielle Hautvorbereitungslösung nach einem der vorhergehenden Ansprüche, wobei der mindestens eine Klebstoff auf (Meth)acrylat-Chemie beruht.

5. Antimikrobielle Hautvorbereitungslösung nach einem der vorhergehenden Ansprüche, wobei der mindestens eine Klebstoff auf Monomeren beruht, die im Wesentlichen frei von wasserlöslichen Monomeren sind.

6. Antimikrobielle Hautvorbereitungslösung nach einem der vorhergehenden Ansprüche, wobei der mindestens eine Klebstoff ein Homopolymer ist.

7. Antimikrobielle Hautvorbereitungslösung nach einem der vorhergehenden Ansprüche, wobei der antimikrobielle Wirkstoff Chlorhexidin in Form freier Base umfasst.

8. Antimikrobielle Hautvorbereitungslösung nach einem der vorhergehenden Ansprüche, wobei der antimikrobielle Wirkstoff ein Chlorhexidinsalz umfasst.

9. Antimikrobielle Hautvorbereitungslösung nach einem der vorhergehenden Ansprüche, wobei in der Hautvorbereitungslösung mindestens 2 Gew.-% Chlorhexidinsalz vorhanden sind.

10. Antimikrobielle Hautvorbereitungslösung nach einem der vorhergehenden Ansprüche, wobei der mindestens eine Klebstoff polare Funktionsgruppen umfasst.

11. Antimikrobielle Hautvorbereitungslösung nach einem der vorhergehenden Ansprüche, wobei der mindestens eine Klebstoff bei Testen gemäß einem hierin beschriebenen 90°-Klebekrafttestverfahren eine Zunahme des Anhaftens von Operationsabdecktüchern und medizinischen Verbandstoffen an mit der antimikrobiellen Hautvorbereitungslösung vorbereiteter Haut von mindestens 10 N/dm vorsieht.

12. Antimikrobielle Hautvorbereitungslösung nach einem der vorhergehenden Ansprüche, wobei der mindestens eine Klebstoff hautkompatibel ist.

13. Applikator, welcher die antimikrobielle Hautvorbereitungslösung nach einem der vorhergehenden Ansprüche in einem oder mehreren Teilen umfasst.

14. Beschichteter Träger, welcher eine reduzierte Hautvorbereitungslösung umfasst, gebildet durch:
Beschichten desselben mit der Hautvorbereitungslösung nach einem der vorhergehenden Ansprüche; und
Trocknen der Beschichtung, um die reduzierte Hautvorbereitungslösung zu bilden, wobei die reduzierte Hautvorbereitungslösung eine flüssige Konsistenz aufweist.

15. Beschichteter Träger nach Anspruch 14, welcher weiterhin einen medizinischen Verbandstoff umfasst, der so daran anhaftet, dass die reduzierte Hautvorbereitungslösung eine Grenzflächen-Haftvermittlerschicht zwischen dem Träger und dem medizinischen Verbandstoff bildet.

## Revendications

1. Solution antimicrobienne de préparation cutanée comprenant :
une quantité majoritaire d'au moins un solvant organique, au moins 80 % en poids sur la base du poids total du solvant comprenant au moins un solvant organique volatil ;
une quantité efficace sur le plan antimicrobien d'au moins un agent antimicrobien contenant de la chlorhexidine ; et
une quantité d'au moins un adhésif efficace pour augmenter l'adhésion de champs opératoires et de pansements médicaux sur de la peau préparée avec la solution antimicrobienne de préparation cutanée,
ledit au moins un adhésif étant distinct dudit au moins un agent antimicrobien et étant liquide à température ambiante.

2. Solution antimicrobienne de préparation cutanée selon la revendication 1, dans laquelle la quantité d'au moins un adhésif efficace pour augmenter l'adhésion de champs opératoires et de pansements médicaux sur de la peau préparée avec la solution antimicrobienne de préparation cutanée va de 1 % à 10 % en poids de la solution de préparation cutanée.

3. Solution antimicrobienne de préparation cutanée selon l'une quelconque des revendications précédentes, dans laquelle ledit au moins un adhésif a une viscosité Brookfield à température ambiante de 400 centiPoise à 1 500 centiPoise.

4. Solution antimicrobienne de préparation cutanée selon l'une quelconque des revendications précédentes, dans laquelle ledit au moins un adhésif est basé sur la chimie des (méth)acrylates.

5. Solution antimicrobienne de préparation cutanée selon l'une quelconque des revendications précédentes, dans laquelle ledit au moins un adhésif est basé sur des monomères qui sont sensiblement exempts de monomères hydrosolubles.

6. Solution antimicrobienne de préparation cutanée selon l'une quelconque des revendications précédentes, dans laquelle ledit au moins un adhésif est un homopolymère.

7. Solution antimicrobienne de préparation cutanée selon l'une quelconque des revendications précédentes, dans laquelle l'agent antimicrobien comprend de la chlorhexidine sous forme de base libre.

8. Solution antimicrobienne de préparation cutanée selon l'une quelconque des revendications précédentes, dans laquelle l'agent antimicrobien comprend un sel de chlorhexidine.

9. Solution antimicrobienne de préparation cutanée selon l'une quelconque des revendications précédentes, dans laquelle au moins 2 % en poids de sel de chlorhexidine sont présents dans la solution de préparation cutanée.

10. Solution antimicrobienne de préparation cutanée selon l'une quelconque des revendications précédentes, dans laquelle ledit au moins un adhésif comprend des groupes fonctionnels polaires.

11. Solution antimicrobienne de préparation cutanée selon l'une quelconque des revendications précédentes, dans laquelle ledit au moins un adhésif apporte une augmentation de l'adhésion de champs opératoires et de pansements médicaux sur de la peau préparée avec la solution antimicrobienne de préparation cutanée d'au moins 10 N/dm lors d'un essai conformément à une méthode d'essai d'adhérence par pelage à 90° ci-décrite.

12. Solution antimicrobienne de préparation cutanée selon l'une quelconque des revendications précédentes, dans laquelle ledit au moins un adhésif est compatible avec la peau.

13. Applicateur comprenant la solution antimicrobienne de préparation cutanée selon l'une quelconque des revendications précédentes dans une ou plusieurs parties.

14. Substrat revêtu comprenant une solution de préparation cutanée réduite formée en :
appliquant la solution antimicrobienne de préparation cutanée sur celui-ci ; et
séchant le revêtement pour former la solution de préparation cutanée réduite, la solution de préparation cutanée réduite ayant une consistance liquide.

15. Substrat revêtu selon la revendication 14, comprenant en outre un pansement médical adhérant sur celui-ci de telle sorte que la solution de préparation cutanée réduite forme une couche de liaison interfaciale entre le substrat et le pansement médical.
